# EUROPÄISCHE PATENTSCHRIFT

(11) **EP 2 603 182 B1**
(45) Veröffentlichungstag und Bekanntmachung des Hinweises auf die Patenterteilung: **09.12.2015**
(21) Anmeldenummer: 11751542.9
(22) Anmeldetag: 08.08.2011
(51) Int. Cl.: A61F 5/01

(54) **ORTHOPÄDIETECHNISCHE FORMTEILANORDNUNG UND VERFAHREN ZUR HERSTELLUNG EINES ORTHOPÄDIETECHNISCHEN FORMTEILS**
ORTHOPAEDIC MOULDING ARRANGEMENT AND METHOD FOR PRODUCING AN ORTHOPAEDIC MOULDING
ENSEMBLE PIÈCE MOULÉE ORTHOPÉDIQUE ET PROCÉDÉ DE FABRICATION D'UNE PIÈCE MOULÉE ORTHOPÉDIQUE

(30) Priorität: 09.08.2010 DE 102010033809
(43) Veröffentlichungstag der Anmeldung: 19.06.2013
(73) Patentinhaber: Otto Bock HealthCare GmbH, 37115 Duderstadt (DE)
(72) Erfinder: KROLL-ORYWAHL, Olaf, 37083 Göttingen (DE); SIEWERT, Gordon, 37077 Göttingen (DE); NOLTE, Michael, 37136 Seeburg (DE); OTTLEBEN, Michael, 37589 Kalefeld (DE)
(74) Vertreter: Friedrich, Andreas
(86) Internationale Anmeldenummer: PCT/EP2011/003959
(87) Internationale Veröffentlichungsnummer: WO 2012/019744

(56) Entgegenhaltungen:
- EP-A2- 0 004 829
- GB-A- 698 255
- US-A- 3 326 211
- US-A- 5 016 624

## Beschreibung

Die Erfindung betrifft ein Verfahren zur Herstellung eines in seiner Form an eine Ausgangsform angepassten Formteils, bei dem mehrere Lagen verformbar so aufeinander gelegt werden, dass sie durch Wärme miteinander verbindbar sind, die Lagen durch Andrücken an die Ausgangsform verformt werden und zur Verbindung der Lagen in dem verformten Zustand Wärme appliziert wird, wobei in die Anordnung aus den Lagen wenigstens ein zugeführte Energie in Wärme umwandelndes Wandlerelement eingebracht wird, mit dem wenigstens Teilbereiche der Lagen zur Verbindung der Lagen aufgeheizt werden, wobei mit der zugeführten Wärme ein themoplastisches Material zum Schmelzen gebracht wird, das anschließend durch Abkühlen erstarrt.

Die Erfindung betrifft ferner eine orthopädietechnische Formteilanordnung mit aufeinandergelegten Lagen, die nach einem Drapieren zur Anpassung an eine Körperform durch Wärmeeinwirkung miteinander verbindbar sind, wobei in die Anordnung aus den Lagen wenigstens ein zugeführte Energie in Wärme umwandelndes Wandlerelement eingebracht ist, mit dem wenigstens Teilbereiche der Lagen kontaktiert sind.

Orthopädietechnische Geräte, wie Prothesen und Orthesen benötigen Formteile, mit denen die Verbindung des Geräts zum Körper hergestellt werden kann. Neben Standardformteilen in verschiedenen Größen werden häufig spezifisch an die betreffende Körperform des Patienten angepasste Formteile verwendet. Die Herstellung dieser spezifisch angepassten Formteile erfolgt in herkömmlicher Weise durch eine Abformung der betreffenden Körperform mittels eines geeigneten Materials, beispielsweise Gips. Diese Abformung wird vom Orthopädietechniker vorgenommen, der die Form regelmäßig in einen Herstellungsbetrieb für orthopädietechnisctie Geräte sendet, um ein orthopädietechnisches Gerät, insbesondere Orthese, einem spezifisch angepassten Formteil herstellen zu lassen. Für ein Formteil einer Orthese ist es dabei beispielsweise erforderlich, mit dem Gipsabdruck eine Positivform herzustellen, die der Form des Körperteils entspricht. Mit dieser Form wird die Formgebung des eigentlichen orthopädietechnischen Formteils vorgenommen. Dieses Verfahren ist nicht nur zeitlich aufwändig, sondern erfordert auch die Herstellung verlorener Formen, die den Preis des orthopädietechnischen Geräts erhöhen.

Durch WO 2008/092443 ist es bekannt, ein orthopädietechnisches Formteil in seiner fertigen Form unmittelbar am Körper des Patienten dadurch auszubilden, dass die miteinander verbindbaren Lagen mit einer plastisch verformbaren Einlage versehen werden, die die Verformung durch die Anpassung (Drapieren) an das betroffene Körperteil beibehält, bis eine Verbindung der Lagen durch die Aushärtung eines Bindemittels abgeschlossen ist. Die Herstellung des Formteils muss dabei unmittelbar nach der Abformung erfolgen, also regelmäßig beim Orthopädietechniker. Dadurch entstehen erhebliche Restriktionen für die Herstellung des orthopädietechnischen Geräts, die in vielen Fällen vorteilhaft in einem spezialisierten Herstellungsbetrieb erfolgt, der über spezialisierte Herstellungseinrichtungen und Prüfeinrichtungen verfügt.

Aus der US 2002/0177797 A1 sind orthopädische Anordnungen bekannt, die aushärtbare Stützelemente umfassen, die an ein Körperteil des Patienten angepasst werden können. Diese flächigen Elemente sind im mittleren Teil mit einem aushärtbaren Material imprägniert, während der Randbereich frei von diesem Material ist. Nach dem Anpassen an den Körper des Patienten wird das Element in herkömmlicher Weise ausgehärtet.

Aus der US 2008/0319362 A1 ist ein System zum Immobilisieren und Abstützen eines Körperteils bekannt, bei dem eine in situ Aushärtung explizit vermieden werden soll.

Die US 6,129,695 offenbart Gelenkschoner, wie sie beispielsweise von Sportlern getragen werden. Derartige Schoner werden beispielsweise mit Kissen oder Schaumpads versehen, um eine bessere Schutzwirkung zu entfalten. Werden die verschiedenen Bestandteile derartiger Gelenkschoner zusammengenäht, können durch die Nähte Irritationen beim Träger auftreten. Um dies zu verhindern, wird vorgeschlagen, die verschiedenen Teile über ein thermoplastisches Material miteinander zu verbinden, das zum Verbinden der Teile aufgeschmolzen wird. Dies geschieht bei der Herstellung der Schoner durch eine Heizelektrode, die auf die zu verbindenden Materialien aufgedrückt wird.

Aus der GB 2 349 822 A ist eine Schiene bekannt, mit der Körperteile geschient und immobilisiert werden können. Diese umfasst eine Hülle, die mit einem thermoplastischen Material gefüllt ist. Zum Anpassen der Schiene an das Körperteil wird das Material erwärmt und somit flüssig. Nach dem Abkühlen verbleibt die Schiene in der an das Körperteil angepassten Form.

Aus der US 3,326,211, der US 5,016,624 und der GB 698255 ist jeweils ein gattungsgemäßes Verfahren bekannt. Die EP 0 044 829 A2 beschreibt eine Zwischenlage zur Anpassung einer nicht dehnbaren Hülle an einen Teil des menschlichen Körpers. Dies kann insbesondere für Skischuhe, Helme oder Boxhandschuhe verwendet werden.

Der vorliegenden Erfindung liegt daher die Aufgabe zugrunde, die Herstellung eines Formteils zu ermöglichen, bei der die benötigte Formstabilität des Formteils beim oder unmittelbar nach dem Abformvorgang von der Ausgangsform in einfacher Weise und mit geringem Aufwand erreichbar ist.

Zur Lösung dieser Aufgabe ist erfindungsgemäß ein Verfahren gemäß Ansprüch 1 vorgesehen.

Zur Lösung der genannten Aufgabe ist eine orthopädietechnische Formteilanordnung gemäß Anspruch 5 vorgesehen.

Erfindungsgemäß ist somit vorgesehen, dass die unter Wärmeeinwirkung miteinander verbindbaren Lagen durch Anpassung an die Ausgangsform, insbesondere an die Körperform, verformt (drapiert) werden und dass nach der Verformung die Verbindung der Lagen miteinander dadurch erfolgt, dass dem Wandlerelement Energie zugeführt wird, mit der die für die Verbindung der Lagen miteinander benötigte Wärmeeinwirkung entsteht.

Ein einfaches Wandlerelement ist ein in die Anordnung der Lagen eingebrachter Heizleiter, der vorzugsweise zwischen wenigstens zwei Lagen angeordnet sein kann. Die Anschlüsse des Heizleiters sind dann aus der Anordnung der Lagen herausgeführt. Durch einen Stromfluss durch den Heizleiter kann die zur Verbindung der Lagen miteinander erforderliche Wärme generiert werden.

In einer anderen Ausführungsform kann in die Anordnung der Lagen eine Mikrowellen absorbierende Anordnung eingelegt sein, die sehr schnell durch eine kurze Mikrowelleneinwirkung aufheizbar und so die für die Verbindung der Lagen erforderliche Wärme generiert. Die Wandlerelemente können ferner durch elektrische Leiter gebildet sein, die mit durch Induktionsspulen erzeugten elektromagnetischen Feldern aufgeheizt werden. In diesem Fall erübrigt sich das Herausführen von Anschlüssen aus der Lagenanordnung. Selbstverständlich sind weitere Wandlerelemente und Anregungen von Wandlerelementen einsetzbar, denen die Energie selektiv zuführbar ist. In Frage kommt beispielsweise auch neben der Hochfrequenzenergie die Zuführung von Ultraschallenergie zur Generierung der benötigten Wärme. Wesentlich für die Erfindung ist dabei, dass die Wärme im Bereich der Wandlerelemente generiert wird und nicht im Material der Lagen selbst. Diese sollten sich allenfalls geringfügig durch die Energiezufuhr erwärmen und ihre für die Verbindung benötigte Energie von den aufgeheizten Wandlerelementen beziehen.

In einer bevorzugten Anwendung der vorliegenden Erfindung wird die Verbindung der Lagen miteinander nur vorläufig hergestellt, insbesondere um die vom Körperteil abgenommene Form sicher zum Herstellungsbetrieb für das orthopädietechnische Gerät transportieren zu können. Die Verbindung der Lagen miteinander ist so stabil, dass die abgenommene Form während des Transports zum Herstellungsbetrieb erhalten bleibt, jedoch in diesem Fall nicht ausreichend stabil ist, um den Belastungen im Gebrauch des orthopädietechnischen Geräts, beispielsweise der Orthese standzuhalten. Demgemäß findet in diesem Fall eine endgültige Verbindung der Lagen miteinander erst im Herstellungsbetrieb für das orthopädietechnische Gerät statt. Daher ist es für diesen Anwendungsfall ausreichend, die Verbindung zwischen den Lagen nur lokal begrenzt, also beispielsweise nur in der unmittelbaren Umgebung eines Heizdrahts oder eines Heizstreifens erfolgt. Eine Verbindung über die gesamte Fläche der Langen ist für diese Zwecke weder erforderlich noch sinnvoll.

Die Verbindung, die zwischen den Lagen durch die Zuführung von Wärme vorgenommen wird, erfolgt mittels eines thermoplastischen Materials. Erfindungsgemäß ist die Verbindung mit eine thermoplastischen Material. Dabei können die Lagen selbst aus einem thermoplastischen Material bestehen oder mit einem thermoplastischen Material beschichtet sein, wobei die Lagen noch separiert voneinander sind. Durch die Zuführung der Wärme kommt es dann zu einer Erweichung des thermoplastischen Materials, wodurch die Lagen in dem Bereich des verflüssigten thermoplastischen Materials miteinander verbunden werden, wenn nach Abkühlung das thermoplastische Material erstarrt. Der gleiche Effekt lässt sich erzielen durch das Einlegen einer thermoplastischen Zwischenlage zwischen die Lagen, beispielsweise in Form einer thermoplastischen Folie.

In einer bevorzugten Ausführungsform sind die Lagen Faserlagen, vorzugsweise Gewebelagen. Dabei können mit Thermoplast getränkte Faserlagen, so genannte Prepregs, zur Anwendung gelangen. Ferner ist es möglich Faserlagen mit Fasern zu verwenden, die einen mit einer thermoplastischen Ummantelung beschichteten Faserkern aufweisen. Bei geeigneter Steuerung der Wärmeeinwirkung können auch massive thermoplastische Fasern verwendet werden, wobei es zweckmäßig ist, dass durch die Wärmeeinwirkung lediglich die Oberfläche der Fasern angeschmolzen wird.

Für die Beibehaltung der durch Andrücken an die Körperform generierten Verformung der Lagen sind die Lagen in an sich bekannter Weise in einer gasdichten Umhüllung angeordnet sein, die vor oder insbesondere nach der Verformung evakuierbar ist. Durch die Ausbildung des Unterdrucks entsteht ein handhabbares Lagenpaket, das gegenüber geringen Belastungen formstabil ist. Dieses evakuierte Lagenpaket kann somit von dem Körperteil abgenommen und dann die erfindungsgemäße Verbindung zwischen den Lagen hergestellt werden. Auf diese Weise gelingt es mit nur lokal angeordneten Wandlerelementen, beispielsweise bereits mit wenigen Windungen eines Heizdrahtes, eine so stabile Verbindung zwischen den Lagen herzustellen, dass das hergestellte Formteil formstabil transportierbar ist, auch wenn sich die Lage nicht (mehr) in einem Vakuum befinden.

Es ist allerdings nicht ausgeschlossen, erfindungsgemäß auch eine endgültige Verbindung der Lagen herbeizuführen, wenn eine entsprechend flächige Aufbringung der Wärme auf die Lagen sichergestellt wird, sodass eine weitgehend flächige Verbindung zwischen den Lagen erfolgt.

Die Erfindung soll im Folgenden anhand von in der Zeichnung dargestellten Ausführungsbeispielen näher erläutert werden. Es zeigen:
- Figur 1: eine schematische Darstellung einer Formteilanordnung zur Herstellung eines an eine Körperform spezifisch angepassten Formteils;
- Figur 2: einen Schnitt entlang der Linie B-B in Figur 1;
- Figur 3: eine schematische Ansicht eines an eine Körperform angepassten Formteils.

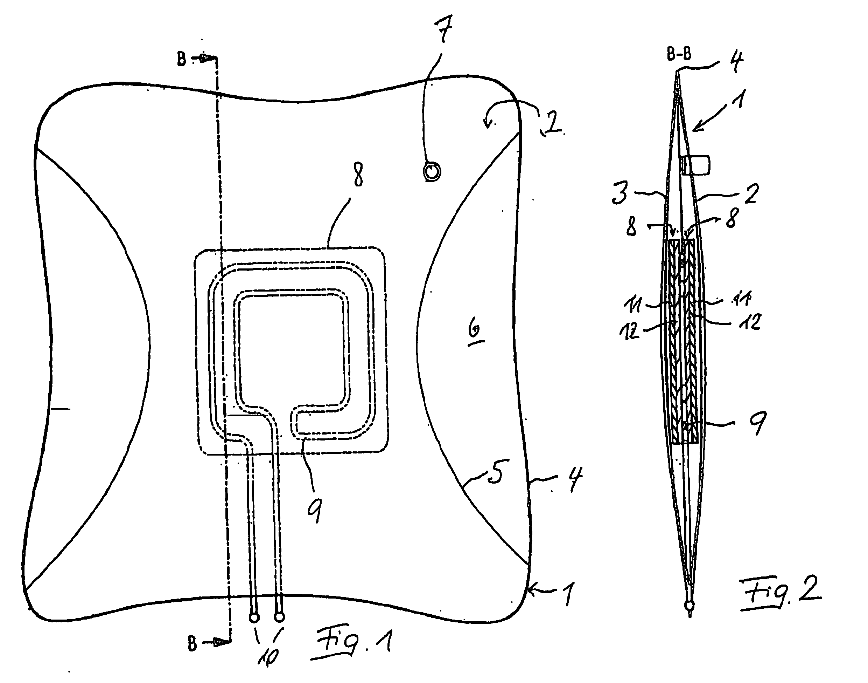

Figur 1 zeigt die Umrisse einer als flachliegender Folienbeutel ausgebildeten Umhüllung 1, die allseitig geschlossen ist. Die Umhüllung ist aus einem gasdichten Material gebildet und besteht im flachliegenden Zustand aus einer Oberseite 2 und einer Unterseite 3, die an einer umlaufenden Kante 4 miteinander verbunden sind. Die umlaufende Kante 4 weist in dem dargestellten Ausführungsbeispiel eine kissenförmige Kontur auf, bei der in Abweichung von einer rechteckigen Grundform mit abgerundeten Ecken in der Mitte der Seitenkanten Taillierungen vorgesehen sind. Auf der Oberseite 2 und der Unterseite 3 ist die Umhüllung auf zwei gegenüberliegenden Seitenkanten mit einer gebogenen Schwächungslinie 5 versehen, die sich auf der jeweiligen Seitenkante von einem Eckbereich zu einem anderen Eckbereich erstreckt. Die Schwächungslinie erlaubt das definierte Abknicken eines durch die bogenförmige Schwächungslinie 5 begrenzten Seitenbereichs 6, um eine dreidimensionale Verformung der Umhüllung 1 zu erleichtern.

Auf der Oberseite 2 der Umhüllung 1 ist ein Ventil 7 vorgesehen, mit dem der durch die Oberseite 2 und die Unterseite 3 begrenzte Innenraum der Umhüllung 1 durch Anschluss an eine Vakuumpumpe evakuierbar ist. Zwischen die Oberseite 2 und die Unterseite 3 der Umhüllung 1 befinden sich mittig in der Umhüllung 1 angeordnet zwei Lagen 8, zwischen denen ein Heizleiter 9 als Wandlerelement eingelegt ist. Der Heizleiter 9 ist in dem dargestellten Ausführungsbeispiel mit mehreren Abbiegungen zu zwei Windungen geformt, um den größten Teil einer Umfangslinie der im Wesentlichen rechteckig ausgebildeten Lagen 8 abzudecken. Der Heizleiter 9 ist mit zwei Anschlüssen 10 aus dem Bereich der Lagen 8 und ferner in gasdichter Form aus der Umhüllung 1 herausgeführt.

Figur 2 verdeutlicht, dass die Lagen 8 jeweils eine äußere Faserlage 11 und eine innere Schicht 12 aus einem thermoplastischen Material aufweisen, sodass der Heizleiter 9 einen unmittelbaren Kontakt zu den beiden Schichten 12 aus thermoplastischem Material hat.

Die Faserlagen 11 können Gewebelagen aus beliebigem Material, beispielsweise Glasfaser, Aramidfasern und/oder Glasfasern sein. Die Schicht 12 kann aus einem beliebigen thermoplastischen Material bestehen, das mit der Faserlage 11 verbindbar ist, sei es durch Verschweißung, Verklebung, Beschichtung o. dgl.

Die in Figur 1 dargestellte Formteilanordnung wird in dieser Form gegen ein abzuformendes Körperteil als Ausgangsform gedrückt, sodass es vollständig und faltenfrei an der betreffenden Körperkontur anliegt. Dann wird über das Ventil 7 aus der Umhüllung die Luft abgesaugt, sodass mit den Lagen 8 und der Umhüllung 1 ein unter Druck aneinanderliegendes Paket gebildet wird, in dem die Lagen 8 sich nicht mehr verformen und nicht mehr gegeneinander bewegen. In diesem Zustand wird zweckmäßigerweise die geformte Formteilanordnung von dem Körperteil abgenommen und eine Stormquelle an die Anschlüsse 10 des Heizleiters 9 angeschlossen. Dadurch werden die Schichten 12 aus thermoplastischem Material der Lagen 8 im Bereich des Heizleiters 9 miteinander verschweißt, da der Stromfluss durch den Heizleiter 9 eine Erwärmung in der Umgebung des Heizleiters 9 bewirkt, durch die das thermoplastische Material lokal schmilzt und sich das geschmolzene thermoplastische Material der gegenüberliegenden Schichten 12 miteinander verbindet, also die Schichten 12 im Bereich des Heizleiters 9 miteinander thermisch verschweißt werden. Da die Schichten 12 mit den Faserlagen 11 verbunden sind, ergibt sich somit ein fester Verbund zwischen den Lagen 8. Die relative Position der Lagen 8 zueinander - und damit auch die angenommene Form des Formteils - sind somit durch die mittels des Heizleiters 9 erzeugten Schweißverbindungen fixiert.

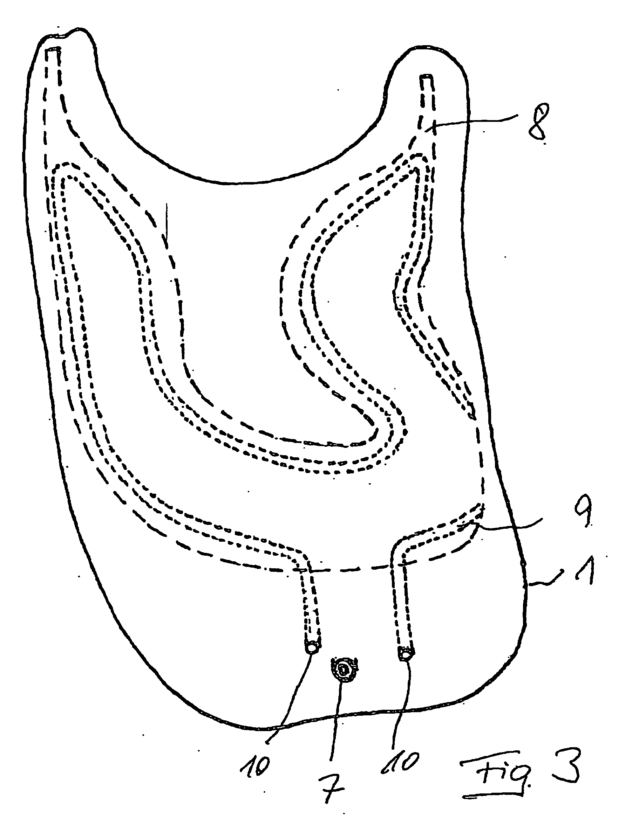

Figur 3 zeigt ein Ausführungsbeispiel eines erfindungsgemäß hergestellten Formteils nach der dreidimensionale Verformung, die durch das Evakuieren der Umhüllung 1 über das Ventil 7 und durch die Verschweißung der Lagen 8 entlang dem Heizleiter 9 fixiert ist. Die Anschlüsse 10 sind aus der Umhüllung 1 in einem nicht störenden Bereich herausgeführt und werden zur Herstellung der endgültigen Form am Rande der Lagen 8 abgeschnitten.

In der Darstellung der Figur 3 befinden sich die Lagen 8, die das Formteil bilden, noch in der Umhüllung 1. In diesem Zustand ist das geformte Teil durch die Umhüllung 1 geschützt transportierbar, wobei es durch die Verschweißung der Lagen 8 miteinander in seiner Form fixiert bleibt. Mit den geformten Lagen 8 kann - nach Abnahme der Umhüllung 1 nach der Durchführung des Transports - das gewünschte orthopädietechnische Gerät hergestellt werden. Hierzu können die Lagen 8 endgültig miteinander verbunden werden, sofern mit dem Heizleiter 9, wie in den Figuren 1 und 2 dargestellt, nur eine vorläufige Verbindung der Lagen 8 durchführt worden ist. Selbstverständlich können die Lagen 8 bei der Herstellung des orthopädietechnischen Geräts auch noch durch weitere Lagen ergänzt werden, die an die Form der Lagen 8 angepasst werden.

Die Erfindung ermöglicht somit die Herstellung eines spezifisch an eine Körperform eines betreffenden Körperteils angepassten orthopädietechnischen Geräts ohne verlorene Formen und in einer optimierten Aufgaben Aufteilung zwischen einem Orthopädietechniker und einem spezialisierten Herstellungsbetrieb für orthopädietechnische Geräte. In analoger Weise können Formteile auch von anderen Ausgangsformen abgeformt hergestellt werden.

## Patentansprüche

1. Verfahren zur Herstellung eines in seiner Form an eine Ausgangsform angepassten Formteils, bei dem mehrere Lagen (8) verformbar so aufeinander gelegt werden, dass sie durch Wärme miteinander verbindbar sind, die Lagen (8) durch Andrücken an die Ausgangsform verformt werden und zur Verbindung der Lagen (8) in dem verformten Zustand Wärme appliziert wird, wobei in die Anordnung aus den Lagen (8) wenigstens ein zugeführte Energie in Wärme umwandelndes Wandlerelement eingebracht wird, mit dem wenigstens Teilbereiche der Lagen (8) zur Verbindung der Lagen (8) aufgeheizt werden, wobei mit der zugeführten Wärme ein thermoplastisches Material zum Schmelzen gebracht wird, das anschließend durch Abkühlen erstarrt, wobei die Lagen (8) aus thermoplastischem Material bestehen oder eine Beschichtung mit thermoplastischem Material aufweisen und in einer gasdichten Umhüllung angeordnet sind, die evakuierbar ist.

2. Verfahren nach Anspruch 1, wobei das Wandlerelement ein Heizleiter (9) ist.

3. Verfahren nach Anspruch 1 oder 2, wobei die Verbindung der verformten Lagen (8) mittels der Wandlerelemente nur vorläufig vorgenommen wird und wobei mit zeitlichem Abstand eine endgültige Verbindung der verformten Lagen (8) erfolgt.

4. Verfahren nach einem der Ansprüche 1 bis 3, wobei die Lagen (8) vor dem Andrücken an die Ausgangsform in eine gasdichte Umhüllung (1) eingebracht werden, und wobei vor oder nach der Verformung der Lagen (8) in der Umhüllung (1) ein Unterdruck erzeugt wird.

5. Orthopädietechnische Formteilanordnung mit aufeinandergelegten Lagen (8), die nach einem Drapieren zur Anpassung an eine Körperform durch Wärmeeinwirkung miteinander verbindbar sind, wobei in die Anordnung aus den Lagen (8) wenigstens ein zugeführte Energie in Wärme umwandelndes Wandlerelement eingebracht ist, mit dem wenigstens Teilbereiche der Lagen (8) kontaktiert sind, wobei die Lagen (8) aus thermoplastischem Material bestehen oder eine Beschichtung aus thermoplastischem Material aufweisen oder wobei zwischen die Lagen (8) thermoplastisches Material eingelegt ist und wobei die Lagen (8) innerhalb einer gasdichten evakuierbaren Umhüllung (1) angeordnet sind.

6. Orthopädietechnische Formteilanordnung nach Anspruch 5, wobei die Verbindung zwischen den Lagen (8) mittels der Wandlerelemente als vorläufige Verbindung konzipiert ist.

7. Orthopädietechnische Formteilanordnung nach einem der Ansprüche 5 oder 6, wobei die Verbindung zwischen den Lagen (8) mittels der Wandlerelemente als endgültige Verbindung konzipiert ist.

8. Orthopädietechnische Formteilanordnung nach einem der Ansprüche 5 bis 7, wobei das Wandlerelement durch Heizleiter (9) gebildet ist.

## Claims

1. Method for producing a moulding, the shape of which is adapted to an initial mould, in which method a plurality of layers (8) are deformably placed one on top of the other in such a way that they can be joined to one another by heat, the layers (8) are deformed by being pressed onto the initial mould and, in order to join the layers (8), heat is applied in the deformed state, wherein at least one converter element which transforms supplied energy into heat and with which at least partial regions of the layers (8) are heated up to join the layers (8) is introduced into the arrangement comprising the layers (8), wherein the supplied heat is used for melting a thermoplastic material, which subsequently solidifies by cooling down, wherein the layers (8) consist of thermoplastic material or have a coating with thermoplastic material and are arranged in a gastight enclosure that can be evacuated.

2. Method according to Claim 1, wherein the converter element is a heating conductor (9).

3. Method according to Claim 1 or 2, wherein the joining of the deformed layers (8) is only temporarily carried out by means of the converter elements, and wherein a permanent joining of the deformed layers (8) occurs after a time interval.

4. Method according to one of Claims 1 to 3, wherein the layers (8) are introduced into a gastight enclosure (1) before being pressed on the initial mould, and wherein a vacuum is created in the enclosure (1) before or after the deformation of the layers (8).

5. Orthopaedic moulding arrangement with layers (8) placed one on top of the other, which can be joined to one another by the effect of heat after having been draped over a body shape for adaptation, wherein at least one converter element which transforms supplied energy into heat and with which at least partial regions of the layers (8) are contacted is introduced into the arrangement comprising the layers (8), wherein the layers (8) consist of thermoplastic material or have a coating with thermoplastic material or wherein thermoplastic material is placed between the layers (8) and wherein the layers (8) are arranged within a gastight enclosure (1) that can be evacuated.

6. Orthopaedic moulding arrangement according to Claim 5, wherein the joining between the layers (8) by means of the converter elements is conceived as a temporary joining.

7. Orthopaedic moulding arrangement according to either of Claims 5 and 6, wherein the joining between the layers (8) by means of the converter elements is conceived as a permanent joining.

8. Orthopaedic moulding arrangement according to one of Claims 5 to 7, wherein the converter element is formed by a heating conductor (9).

## Revendications

1. Procédé pour la fabrication d'une pièce moulée adaptée quant à sa forme à une forme de départ, dans lequel plusieurs couches (8) sont posées les unes sur les autres de façon déformable de telle manière qu'elles peuvent être reliées les unes aux autres au moyen de chaleur, les couches (8) sont déformées par pressage contre la forme de départ et on applique de la chaleur pour relier les couches (8) dans l'état déformé, dans lequel on introduit dans l'agencement composé des couches (8) au moins un élément convertisseur qui convertit en chaleur de l'énérgie apportée et au moyen duquel au moins des zones partielles des couches (8) sont chauffées pour relier les couches (8), dans lequel au moyen de la chaleur apportée on amène en fusion un matériau thermoplastique qui se fige ensuite par refroidissement, dans lequel les couches (8) sont en matériau thermoplastique ou comprennent un revêtement en matériau thermoplastique et sont agencées dans une enveloppe étanche aux gaz qui est susceptible d'être évacuée.

2. Procédé selon la revendication 1, dans lequel l'élément convertisseur est un conducteur chauffant (9).

3. Procédé selon la revendication 1 ou 2, dans lequel la liaison des couches déformées (8) au moyen des éléments convertisseurs n'est effectuée que provisoirement, et dans lequel une liaison définitive des couches déformées (8) a lieu avec un écart temporel.

4. Procédé selon l'une des revendications 1 à 3, dans lequel les couches (8) sont introduites dans une enveloppe étanche aux gaz (1) avant d'être pressées contre la forme de départ, et dans lequel on engendre une dépression dans l'enveloppe (1) avant ou après la déformation des couches (8).

5. Agencement technique constituant une pièce moulée orthopédique comprenant des couches posées les unes sur -les autres (8) qui, après avoir été drapées pour s'adapter à une forme corporelle, peuvent être reliées les unes aux autres par action de chaleur, dans lequel au moins un élément convertisseur qui convertit en chaleur une énergie apportée est introduit dans l'agencement composé des couches (8), élément avec lequel au moins des zones partielles des couches (8) sont mises en contact, dans lequel les couches (8) sont en matériau thermoplastique ou comprennent un revêtement en matériau thermoplastique, ou dans lequel un matériau thermoplastique est introduit entre les couches (8), et dans lequel les couches (8) sont agencées à l'intérieur d'une enveloppe (1) étanche aux gaz susceptible d'être évacuée.

6. Agencement technique constituant une pièce moulée orthopédique selon la revendication 5, dans lequel la liaison entre les couches (8) au moyen des éléments convertisseurs est conçue comme une liaison provisoire.

7. Agencement technique constituant une pièce moulée orthopédique selon l'une des revendications 5 au 6, dans lequel la liaison entre les couches (8) au moyen des éléments convertisseurs est conçue comme une liaison définitive.

8. Agencement technique constituant une pièce moulée orthopédique selon l'une des revendications 5 à 7, dans lequel l'élément convertisseur est formé par des conducteurs chauffants (9).
